# EUROPÄISCHE PATENTSCHRIFT

(11) **EP 1 790 327 B1**
(45) Veröffentlichungstag und Bekanntmachung des Hinweises auf die Patenterteilung: **19.07.2017**
(21) Anmeldenummer: 06021561.3
(22) Anmeldetag: 13.10.2006
(51) Int. Cl.: A61Q 19/00, A61K 8/39, A61K 8/49, A61Q 5/00, A61K 8/06

(54) **Flüssige, PEG-freie, kalt verarbeitbare Öl-in-Wasser-Emulgatoren, erhältlich durch Kombination von Emulgatoren auf der Basis von Polyolpartialestern und Zitronensäurepartialestern**
Liquid, PEG-free O/W emulifiers, for use in ambient temperature, obtainable by a combination of emuslifiers on the basis of partial esters of polyols and partial esters of citric acid
Emulsifiants pour la préparation d'emulsions H/E, fluides et dépourvues de PEG, obtenues par combinaison d'esters partiels de polyols et d'acide citrique.

(30) Priorität: 26.10.2005 DE 102005051222
(43) Veröffentlichungstag der Anmeldung: 30.05.2007
(73) Patentinhaber: Evonik Degussa GmbH, 45128 Essen (DE)
(72) Erfinder: Allef, Petra, 53121 Bonn (DE); Hameyer, Peter, 45138 Essen (DE); Meyer, Jürgen, 48143 Münster (DE)

(56) Entgegenhaltungen:
- EP-A- 1 250 916
- WO-A-2004/112731
- DE-A1- 19 837 841
- ABE YOSHIKO ET AL: "Manufacture of milk white oil-in-water emulsions for cosmetics and pharmaceuticals" CHEMICAL ABSTRACTS + INDEXES, AMERICAN CHEMICAL SOCIETY. COLUMBUS, US, Bd. 134, Nr. 17, 2001, Seite 1182, XP002182434 ISSN: 0009-2258
- 'Polyglyceryl-10 Laurate MSDS', [Online] 28 Mai 2013, XP055064308 Gefunden im Internet: <URL:http://www.barnetproducts.com/pdfs/msd s/Barsolve NS-100.pdf> [gefunden am 2013-05-28]
- 'GPS Safety Summary - Sorbitan Laurate', [Online] 28 Mai 2013, XP055064310 Gefunden im Internet: <URL:http://corporate.evonik.de/_layouts/We bsites/Internet/DownloadCenterFileHandler.a shx?fileid=1177> [gefunden am 2013-05-28]

## Beschreibung

Die Erfindung betrifft flüssige PEG-freie kalt-verarbeitbare Öl-in-Wasser-Emulgatorkombinationen enthaltend flüssige Emulgatoren auf der Basis von Polyolpartialestern, bevorzugt Sorbitan- oder Polyglycerinpartialestern und zumindest teilweise neutralisierten Säurepartialestern, bevorzugt Fruchtsäurepartialestern, und gegebenenfalls der zur Erreichung einphasiger Systeme notwendigen Menge polarer Lösungsvermittler, vorzugsweise Wasser. Die Erfindung betrifft weiterhin die Verwendung dieser Emulgatoren zur Herstellung kosmetischer, pharmazeutischer und technischer Formulierungen, insbesondere von Emulsionen, sowie ferner ein Verfahren zur Herstellung und Stabilisierung von Öl-in-Wasser-Emulsionen unter Benutzung der erfindungsgemäßen Emulgatorkombinationen.

An Öl-in-Wasser-Emulgatoren, die auf nativen Rohstoffen basieren, besteht aus ökologischen Gründen sowohl bei den Produzenten als auch bei den Konsumenten von Emulsionspräparaten ein erhebliches Interesse. Vorzugsweise handelt es sich dabei um Emulgatoren, die keine polyethylenglykolhaltigen Reste enthalten ("PEG-freie" Emulgatoren). Aus diesem Grunde finden Partialester aus Polyalkoholen, wie Glycerin, Polyglycerin, Sorbitol oder Methylglykosid und Fettsäuren, wie Laurin-, Öl- oder Isostearinsäure, eine vielfältige Verwendung. Viele dieser Emulgatoren sind trüb und neigen zur Separation.

Nachteil an allen erwähnten Emulgatoren ist die Tatsache, dass sie von pastöser bis fester Konsistenz sind und deshalb vor der Anwendung aufgeschmolzen werden müssen. In Zeiten, in denen Prozessabläufe optimiert und Energiekosten begrenzt werden müssen, bedeutet dies einen deutlichen Wettbewerbsnachteil gegenüber Emulgatoren, die bei Raumtemperatur flüssig sind und somit ohne vorheriges Aufheizen direkt verarbeitet werden können.

Die EP-A-1 250 916 beschreibt eine Mischung aus Polyglycerinpartialestern und Sorbitanpartialestern, die kaltverarbeitbar ist. Beschrieben werden Polyglycerinpartialester aus C₈- bis C₁₈-, vorzugsweise C₁₂-, Fettsäure mit Polyglycerin-5 bis Polyglycerin-15, vorzugsweise Polyglycerin-10, und Sorbitanpartialester aus C₈- bis C₁₈-, vorzugsweise C₁₂-, Fettsäure und Sorbitol. Diese Emulgatoren sind allerdings nicht universell einsetzbar und bilden oftmals keine lagerstabilen Emulsionen.

Die JP-A-60-262827 beschreibt eine flüssige Mischung zur Emulgierung von Siliconölen bestehend aus C₁₂₋₁₈-Sorbitanpartialestern, C₁₂₋₁₈-Polyglycerinpartialestern und Siliconölen.

Die EP-B-0 853 494 beschreibt die Verwendung von Gemischen aus einem Öl-in-Wasser-Emulgator (Alkylpolyglucosiden und/oder Fettsäure-N-alkylpolyhydroxyalkylamiden) und einem Wasser-in-Öl-Emulgator (Polyolpolyhydroxystearaten) als flüssige, leicht pumpbare Öl-in-Wasser-Emulgatoren. Um gut pumpbare Systeme zu erhalten wird der Emulgator mit Polyolen, vorzugsweise Glycerin, verdünnt.
Das gleiche hydrophile Emulgatorsystem wird in DE-A-103 34 225 und DE-A-103 46 515 in Kombination mit Alkylacylglutamaten zur Herstellung von Nanoemulsionen beschrieben.

Die DE-A-198 37 841 beschreibt die Verwendung von wässrigen Dispersionen von Emulgator und Wachskörper zur Kaltherstellung von Emulsionen. Die Herstellung der Dispersionen erfolgt allerdings bei der Schmelztempertaur des Waches. Es werden keine Aussagen über die Lagerstabilität der Dispersionen gemacht. In der Regel müssen diese Dispersionen immer frisch angesetzt werden, was zumindest den Zeitvorteil insgesamt minimiert.

Die im Stand der Technik beschriebenen kaltverarbeitbaren PEG-freien Emulgatorsysteme führen meist nur zu einer mäßigen Emulsionsstabilisierung, was in Abhängigkeit des zu emulgierenden Systems zu Einschränkungen führt, die einer universellen Verwendung dieser Systeme entgegensteht.

Eine Aufgabe der Erfindung bestand daher darin, PEG-freie Öl-in-Wasser-Emulgatoren zu entwickeln, die bei Raumtemperatur flüssig sind und damit zur Kaltherstellung von Emulsionen mit sehr guter Emulsionsstabilität eingesetzt werden können, wodurch sich wesentlich erweiterte Verwendungsmöglichkeiten dieser Systeme ergeben.

Überraschenderweise wurde nun gefunden, dass flüssige, PEG-freie Polyolpartialester, die entweder Öl-in-Wasser-Emulgatoren mit einer alleine unzureichenden Emulgieraktivität oder sogar Wasser-in-Öl Emulgatoren sind, durch Zusatz geringer Mengen von Emulgatoren, die eine zumindest teilweise neutralisierte Säurefunktion tragen, in leistungsstarke, kaltverarbeitbare Öl-in-Wasser-Emulgatoren überführt werden können.

Die Leistungstärke dieser neuartigen, kaltverarbeitbaren PEG-freien Emulgatoren zeigt sich in Ihrer breiten Anwendungsfähigkeit zur Emulgierung verschiedenster kosmetischer Öle, sowie in den geringen Emulgatorkonzentrationen (1 bis 3 Gew.-% bezogen auf Gesamtemulsion), die zur Bildung stabiler Emulsionen benötigt werden.

Dabei lassen sich die benötigten Mengen der zugesetzten Emulgatoren mit zumindest teilweise neutralisierten Säurefunktionen gegebenenfalls durch Zusatz polarer Lösungsvermittler (im einfachsten Fall Wasser) derart in die flüssige Emulgatorgrundlage einarbeiten, dass ein einphasiges, vorzugsweise klares System entsteht. Als besonders geeignet hat sich ein Gemisch bestehend aus 70 bis 90 Gew.-% Sorbitanester, 8 bis 20 Gew.-% hydrophiler Polyglycerinester und 2 bis 10 Gew.-% neutralisierter Zitronensäurepartialester erwiesen. Dabei werden die besten Ergebnisse erhalten, wenn als hydrophober Rest in allen drei Emulgatorkomponenten Laurinsäure zum Einsatz kommt.

Ein Gegenstand der Erfindung sind somit flüssige, kalt verarbeitbare, einphasige Öl-in-Wasser-Emulgatorsysteme, enthaltend eine flüssige, PEG-freie Emulgatorbasis bestehend aus
A) einem oder mehreren Polyolpartialestern ausgewählt aus mindestens einer der Gruppen
   A1) Sorbitan- oder Sorbitolpartialester, vorzugsweise herstellbar durch Veresterung von aliphatischen, linearen oder verzweigten, ggf. ungesättigten und/oder hydroxyfunktionalisierten Carbonsäuren mit einer Kettenlänge von 6 bis 22 C-Atomen mit Sorbitol,
   A2) Glycerin- und Polyglycerinpartialester, vorzugsweise herstellbar durch Veresterung von aliphatischen, linearen oder verzweigten, ggf. ungesättigten und/oder hydroxyfunktionalisierten Carbonsäuren mit einer Kettenlänge von 6 bis 22 C-Atomen mit Glycerin, Polyglycerinen oder Gemischen aus beiden, und
B) neutralisierte Zitronensäurepartialester, deren hydrophobe Reste jeweils 10 bis 16 C-Atome enthalten und gegebenenfalls
C) polaren flüssigen Lösungsvermittlern.

Üblicherweise besteht der Hauptteil der flüssigen Polyolpartialesterkomponente (A) aus Sorbitanestern, denen vorzugsweise hydrophile Polyglycerinester in einer Menge von 0 bis 30 Gew.-%, vorzugsweise 3 bis 25 Gew.-%, besonders bevorzugt 8 bis 20 Gew.-% beigemischt werden.
Bevorzugt ist dabei eine Kombination auf Basis von Sorbitan- und Polyglycerinpartialestern, die als hydrophobe Komponenten Fettsäurereste mit einer Kettenlänge von 10 bis 16 C-Atomen enthalten.

Ganz besonders bevorzugt ist dabei eine Kombination aus Sorbitan-Lauraten und Polyglyceryl-Lauraten.

Die Emulgatoren des Typs (B) werden, bezogen auf die aus Polyolpartialestern bestehende flüssige Emulgatorbasis, in Mengen von maximal 20 Gew.-%, bevorzugt ≤ 10 Gew.-%, besonders bevorzugt ≤ 5 Gew.-% eingesetzt.

Ganz besonders bevorzugt sind dabei die Partialester aus Zitronensäure und Laurylalkohol bzw. aus Zitronensäure und Glyceryl Mono- oder Dilauraten.

Als flüssige polare Lösungsvermittler zur Herstellung einphasiger, möglichst klarer erfindungsgemäßer kaltverarbeitbarer Öl-in-Wasser-Emulgatorsysteme können vorzugsweise Wasser, Glykole, Polyalkylenglykole, Glycerin, Polyglycerine, Alkohole in einer Gesamtmenge von höchstens 20 Gew.-%, vorzugsweise höchstens 10 Gew.-% des gesamten Emulgatorsystems verwendet werden. Die Menge der benötigten polaren Lösungsvermittler ist im Wesentlichen abhängig vom Emulgatortyp und Emulgatorverhältnis und kann durch Handversuche einfach ermittelt werden. In der Regel reichen Mengen von 2 bis 10 Gew.-% Lösungsvermittler aus.

Ein weiterer Gegenstand der Erfindung ist die Verwendung der erfindungsgemäßen Öl-in-Wasser-Emulgatorsysteme zur Herstellung kosmetischer, dermatologischer oder pharmazeutischer Zubereitungen. Bei diesen Zubereitungen handelt es sich vorzugsweise um Emulsionen, die gegebenenfalls auch dispergierte Feststoffe enthalten können.

Ein weiterer Gegenstand der Erfindung ist die Verwendung der erfindungsgemäßen Öl-in-Wasser-Emulgatorsysteme zur Herstellung von Pflege- und Reinigungsmitteln für Haushalt und Industrie, insbesondere für harte Oberflächen, Leder oder Textilien. Vorzugsweise handelt es sich bei diesen Zubereitungen um Emulsionen, die gegebenenfalls auch dispergierte Feststoffe enthalten können.

Bevorzugt ist der Einsatz der erfindungsgemäßen Öl-in-Wasser-Emulgatoren zur Kaltherstellung von kosmetischen Formulierungen zur Pflege und Reinigung von Haut und Haar.

Dabei kann es sich beispielsweise um Cremes oder Lotionen zur Hautpflege, um Produkte auf tensidischer Basis zur Reinigung und Pfege von Haut und Haar, um Sonnenschutzprodukte, um pigmenthaltige Produkte aus dem Bereich der dekorativen Kosmetik (z.B. Make Up, Produkte für Lid/Wimperncolorierung), um Produkte zur Konditionierung von Haar, um Nagelpflegeprodukte oder um Antitranspirantien/Deodorantien handeln.

In diesen Formulierungen können die erfindungsgemäßen Öl-in-Wasser-Emulgatoren zusammen mit dem Fachmann als Stand der Technik bekannten Hilfs- und Zusatzstoffen wie Ölen und Wachsen, Co-Tensiden und Co-Emulgatoren, Konsistenzgebern, Verdickungsmitteln z.B. auf Polymerbasis, UV-Lichtschutzfiltern, Selbstbräunern, Antioxidantien, Hydrotropen, Deodorant- und Antitranspirantwirkstoffen, Wirkstoffen, Farbstoffen, Konservierungsmitteln und Parfüms eingesetzt werden.

Als Wirkstoffe sind dabei insbesondere Tocopherol, Tocopherolacetat, Tocopherolpalmitat, Ascorbinsäure, Desoxyribonucleinsäure, Coenzym Q10, Retinol- und Retinylderivate, Bisabolol, Allantoin, Phytantriol, Panthenol, AHA-Säuren, Aminosäuren, Hyaluronsäure, Creatin (und Creatinderivate), Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Pflanzenextrakte und Vitaminkomplexe bevorzugt.

Die besonders bevorzugten, kaltherstellbaren Öl-in-Wasser-Emulsionen können insbesondere durch Zusatz bekannter Polymerverdicker stabilisiert werden, wie z.B. Polysaccharide, insbesondere Xanthan-Gum, Guar und Guarderviate, Agar-Agar, Alginate und Tylosen, Cellulose und Cellulosederivate, wie z.B. Carboxymethylcellulose, Hydroxyethylcellulose, Hydroxymethylpropylcellulose, außerdem alkylmodifizierte Zuckerderivate wie z.B. Cetylhydroxyethylcellulose. Besonders bevorzugt werden Carbomere (vernetzte Polyacrylate) eingesetzt, die auch alkylmodifiziert sein können, weiterhin Polyacrylamide oder Copolymere, die beispielsweise aus Komponenten wie Acrylsäure, Methacrylsäure, Acrylamid oder Acrylamidopropansulfonsäure aufgebaut sein können, ferner auch Polyvinylalkohol und Polyvinylpyrrolidon.
Aus dem bekannten Stand der Technik war nicht ableitbar, dass man durch Zusatz geringer Mengen neutralisierter Fruchtsäurepartialester aus einem schwach- bis nichtaktiven Öl-in-Wasser-Emulgiersystem einen höchst effizienten, breit verwendbaren, kalt verarbeitbaren Öl-in-Wasser-Emulgator machen kann.

In den Ausführungsbeispielen wird belegt, dass ein Emulgator wie Sorbitan Mono/Di/Triester (INCI: Sorbitan Laurate), dessen Emulgieraktivität zur Herstellung stabiler Öl-in-Wasser-Emulsionen bekanntermaßen nicht ausreicht, durch Kombination mit neutralisierten Zitronensäurepartialestern in einen kaltverarbeitbaren PEG-freien Emulgator mit gutem Stabilisierungspotential überführt werden kann.
Weiterhin wird belegt dass eine zur Herstellung von Öl-in-Wasser-Emulsionen nicht geeignete Emulgatormischung aus Sorbitanestern und Polyglycerinpartialestern durch Kombination mit neutralisierten Zitronensäurepartialesten ebenfalls in einen hocheffektiven, kaltverarbeitbaren PEG-freien Emulgator überführt werden kann.

Die erfindungsgemäße Kombination von Polyolpartialestern insbesondere mit Zitronensäurepartialestern, deren gute Bioabbaubarkeit aus dem Nahrungsmittelsektor bekannt ist, stellt eine auch unter ökologischen Gesichtspunkten vorteilhafte Emulgatormischung mit einer breiten Anwendungsvielfalt zur Verfügung. Durch den erfindungsgemäßen Einsatz zur Kaltherstellung von Emulsionen ist darüber hinaus ein ressourcenschonender Umgang mit dem Faktor Energie gewährleistet.

In diesem praxisrelevanten, ökonomisch und ökologisch höchst vorteilhaften Kernaspekt der vorliegenden Erfindung zeigen sich deutlich deren enormen Vorteile bei Herstellung und Stablisierung kaltverarbeitbarer, PEG-freier Öl-in-Wasser-Emulsionen.

### Ausführungsbeispiele:

Die folgenden Beispielemulsionen sollen den Gegenstand der Erfindung näher erläutern, ohne ihn auf diese Beispiele einzuschränken.
Die Konzentrationsangaben in allen Beispielen sind als Gew.-% angegeben.

Die Emulsionen wurden so hergestellt, dass Öl- und Wasserphase bei Raumtemperatur vereinigt wurden. Anschließend erfolgte die Homogenisierung. Der pH-Wert der Emulsionen wurde auf 5,5 bis 6 eingestellt.
Als Stabilisatoren wurde eine übliche Kombination aus Xanthan Gum und Carbomer verwendet, die als ölige Dispersion zur Ölphase zugesetzt wurden.

Für die Beispiele wurden Formulierungen mit lotionsartiger Konsistenz ausgewählt.

Die Beispiele zeigen insbesondere, wie die Verwendung einer kaltverarbeitbaren Emulgatormischung, die erfindungsgemäße Mengen einer teilneutralisierten, Säuregruppen enthaltenden Emulgatorkomponente (B) enthält, es ermöglicht Öl-in-Wasser-Emulsionen mit guter bis sehr guter Stabilität zu erhalten.

In den Vergleichsbeispielen wird gezeigt, dass keine stabilen Emulsionen erhalten werden, wenn auf Zusatz der erfindungsgemäßen Emulgatorkomponente (B) verzichtet wird und lediglich die flüssige Basiskomponente (A) verwendet wird.

Somit illustrieren diese Beispiele die technische Lehre dieser Anmeldung, welche zeigt, wie durch erfindungsgemäße Kombination zweier - alleine zur Kaltherstellung von Emulsionen nicht geeigneter - Emulgatorkomponenten hocheffektive, vielseitig verwendbare kaltverarbeitbare Öl-in-Wasser-Emulgatoren erhalten werden könnnen.

Um die vielseitige Verwendbarkeit der erfindungsgemäßen Emulgatorsysteme zu demonstrieren wurde bei der Auswahl der eingesetzten Öle meist auf ein Emollient mit hoher Polarität (Capryl/Caprin-Triglyceride) und ein Emollient mit sehr niedriger Polarität (Paraffinum Perliquidum) zurückgegriffen. Teilweise wurde auch ein Emollient mit mittlerer Polarität (Ethylhexyl-Palmitat) verwendet.

Bei der Beurteilung der Stabilität der Emulsionen bedeutet instabil, dass solche Systeme während der Lagerzeit/Lagerbedingungen (3 Monate bei Raumtemperatur und 45 °C; drei Gefrier-Tau-Zyklen zwischen Raumtemperatur und -15 °C) Wasser- oder Ölseparation zeigten.

Sehr gute Stabilität heißt dabei, dass diese Emulsionen keinerlei Instabilitäten zeigten, wie etwa Anzeichen für Phasenseparation, Veränderungen von Viskosität oder Dispersionsgrad.

Für Emulsionen mit guter Stabilität gilt das Gleiche mit der Einschränkung, dass nach dem Kältetest eine graduelle Abnahme der Weißfärbung der Emulsionen zu beobachten war, jedoch in keinem Falle eine Wasser- oder Ölseparation.

Beschreibung der in den Beispielformulierungen eingesetzten Emulgatorsysteme (die Gesamtprozentzahl pro Emulgatorsystem addiert sich jeweils auf 100):
Emulgator 1:

| | |
|---|---|
| Emulgatorkomponente A: | 85 % Sorbitan Laurate¹⁾ |
| | 8 % Polyglyceryl-4 Laurate²⁾ |
| Emulgatorkomponente B: | 3 % Potassium Di-Glycerylmonolaurate Citrate |
| Polarer Lösungsvermittler: | 4 % Wasser |

| | |
|---|---|
| ¹⁾ TEGO^{®} SML (Degussa) ²⁾ TEGO^{®} Care PL 4 (Degussa) | |

### Vergleichsemulgator 1:

Ausschschließlich Emulgatorkomponente A (zu 100 %, d.h. 91,4 % Sorbitan Laurate und 8,6 % Polyglyceryl-4 Laurate).
Bei Potassium Di-Glycerylmonolaurate Citrate handelt es sich um das Kaliumsalz des Diesters aus Zitronensäure und Glycerylmonolaurate.

### Emulgator 2:

| | |
|---|---|
| Emulgatorkomponente A: | 85 % Sorbitan Laurate¹⁾ |
| | 8 % Polyglyceryl-4 Laurate²⁾ |
| Emulgatorkomponente B: | 3 % Potassium Di-Glycerylmonostearate Citrate |
| Polarer Lösungsvermittler: | 4 % Wasser |

Bei Potassium Di-Glycerylmonostearate Citrate handelt es sich um das Kaliumsalz des Diesters aus Zitronensäure und Glycerylmonostearate.

### Emulgator 3:

| | |
|---|---|
| Emulgatorkomponente A : | 85 % Sorbitan Laurate¹⁾ |
| | 8 % Polyglyceryl-4 Laurate²⁾ |
| Emulgatorkomponente B: | 3 % Potassium Dilauryl Citrate |
| Polarer Lösungsvermittler: | 4 % Wasser |

Bei Potassium Dilauryl Citrate handelt es sich um das Kaliumsalz des Diesters aus Zitronensäure und Laurylalkohol.

### Emulgator 4:

| | |
|---|---|
| Emulgatorkomponente A: | 80 % Sorbitan Laurate¹⁾ |
| | 14 % Polyglyceryl-10 Laurate |
| Emulgatorkomponente B: | 2 % Potassium Di-Glycerylmonolaurate Citrate |
| Polarer Lösungsvermittler: | 4 % Wasser |

### Vergleichsemulgator 2:

Ausschschließlich Emulgatorkomponente A (zu 100 %; d.h. 85 % Sorbitan Laurate und 15 % Polyglyceryl-10 Laurate)
Emulgator 5:

| | |
|---|---|
| Emulgatorkomponente A: | 95 % Sorbitan Laurate¹⁾ |
| Emulgatorkomponente B: | 3 % Potassium Di-Glycerylmonolaurate Citrate |
| Polarer Lösungsvermittler: | 2 % Wasser |

### Vergleichsemulgator 3:

Ausschschließlich Emulgatorkomponente A (d.h. 100 % Sorbitan Laurate)
Emulgator 6:

| | |
|---|---|
| Emulgatorkomponente A: | 98 % Polyglyceryl-4 Laurate²⁾ |
| Emulgatorkomponente B: | 0,3 % Potassium Di-Glycerylmonolaurate Citrate |
| | 0,2 % Sodium Lauryl Sulfate |
| Polarer Lösungsvermittler: | 1,5 % Wasser |

Emulgator 7:

| | |
|---|---|
| (nicht erfindungsgemäß) | |
| Emulgatorkomponente A: | 80 % Sorbitan Laurate |
| | 12 % Polyglyceryl-4 Laurate |
| Emulgatorkomponente B: | 3 % Sodium Lauryl Sulfate |
| Polarer Lösungsvermittler: | 5 % Wasser |
| Emulgator 8: | |
| Emulgatorkomponente A: | 64 % Sorbitan Oleate³⁾ |
| | 28 % Polyglyceryl-4 Caprate⁴⁾ |
| Emulgatorkomponente B: | 3 % Potassium Di-Glycerylmonostearate Citrate |
| Polarer Lösungsvermittler: | 5 % Wasser |

| | |
|---|---|
| ³⁾ TEGO^{®} SMO V (Degussa) ⁴⁾ TEGOSOFT^{®} PC 41 (Degussa) | |

| | | 1 | 2 | 3 | 4 | 5 | V1 (vgl. 1, 3, 5) | V2 (vgl. 2, 4) |
|---|---|---|---|---|---|---|---|---|
| A | Emulgator 1 | 1,50 % | 1,50 % | | | | | |
| | Vergleichsemulgator 1 | | | | | | 1,50 % | 1,50 % |
| | Emulgator 2 | | | 1,50 % | 1,50 % | | | |
| | Emulgator 3 | | | | | 1,50 % | | |
| | Capryl/Caprin-Triglyceride | | 18,50 % | | 18,50 % | | | 18,50 % % |
| | Paraffinum Perliquidum | 18,50 % | | 18,50 % | | 18,50 % | 18,50% | |
| | Ethylhexyl-Palmitate | 1,10 % | 1,10 % | 1,10 % | 1,10 % | 1,10 % | 1,10 % | 1,10 % |
| | Xanthan Gum | 0,20 % | 0,20 % | 0,20 % | 0,20 % | 0,20 % | 0,20 % | 0,20 % |
| | Carbomer | 0,20 % | 0,20 % | 0,20 % | 0,20 % | 0,20 % | 0,20 % | 0,20 % |
| B | Glycerin | 2,35 % | 2,35 % | 2,35 % | 2,35 % | 2,35 % | 2,35 % | 2,35 % |
| | Wasser | ad 100 % | ad 100 % | ad 100 % | ad 100 % | ad 100 % | ad 100 % | ad 100 % |
| C | NaOH (10 %) | q.s. | q.s. | q.s. | q.s. | q.s. | q.s. | q.s. |
| | Preservative, Parfüm | q.s. | q.s. | q.s. | q.s. | q.s. | q.s. | q.s. |
| | Stabilität | sehr gut | sehr gut | sehr gut | sehr gut | sehr gut | Instabil | Instabil |

| | | 6 | 7 | 8 | 9 | V3 (vgl. 6 | V4 (vgl. 7) | V5 (vgl. 8) |
|---|---|---|---|---|---|---|---|---|
| A | Emulgator 4 | 1,50 % | 1,50 % | | | | | |
| | Emulgator 5 | | | 1,50 % | | | | |
| | Emulgator 6 | | | | 1,50 % | | | |
| | Vergleichsemulgator 2 | | | | | 1,50 % | 1,50 % | |
| | Vergleichsemulgator 3 | | | | | | | 1,50 % |
| | Capryl/Caprin-Triglyceride | | 18,50 % | | 18,50 % | | 18,50 % | |
| | Paraffinum Perliquidum | 18,50 % | | 18,50 % | | 18,50 % | | 18,50 % |
| | Ethylhexyl-Palmitate | 1,10 % | 1,10 % | 1,10 % | 1,10 % | 1,10 % | 1,10 % | 1,10 % |
| | Xanthan Gum | 0,20 % | 0,20 % | 0,20 % | 0,20 % | 0,20 % | 0,20 % | 0,20 % |
| | Carbomer | 0,20 % | 0,20 % | 0,20 % | 0,20 % | 0,20 % | 0,20 % | 0,20 % |
| B | Glycerin | 2,35 % | 2,35 % | 2,35 % | 2,35 % | 2,35 % | 2,35 % | 2,35 % |
| | Wasser | ad 100 % | ad 100 % | ad 100 % | ad 100 % | ad 100 % | Ad 1,50 % | ad 100 % |
| C | NaOH (10 %) | q.s. | q.s. | q.s. | q.s. | q.s. | q.s. | q.s. |
| | Preservative, Parfüm | q.s. | q.s. | q.s. | q.s. | q.s. | q.s. | q.s. |
| | Stabilität | sehr gut | sehr gut | gut | sehr gut | Instabil | Instabil | Instabil |

| | | 10 | 11 | 12 | 13 | 14 | 15 | V6 (vgl. 10, 11, 12) |
|---|---|---|---|---|---|---|---|---|
| A | Emulgator 1 | 1,50 % | | | | | | |
| | Emulgator 2 | | 1,50 % | | | | | |
| | Emulgator 3 | | | 1,50 % | | | | |
| | Vergleichs emulgator 1 | | | | | | | 1,50 % |
| | Emulgator 7 | | | | 1,50 % | | | |
| | Emulgator 8 | | | | | 1,50 % | 1,50 % | |
| | Capryl/Caprin-Triglyceride | | | | | | 18,50 % | |
| | Paraffinum Perliquidum | | | | | 18,50 % | | |
| | Ethylhexyl-Palmitate | 19,60 % | 19,60 % | 19,60 % | 19,60 % | 1,10 % | 1,10 % | 19,60 % |
| | Xanthan Gum | 0,20 % | 0,20 % | 0,20 % | 0,20 % | 0,20 % | 0,20 % | 0,20 % |
| | Carbomer | 0,20 % | 0,20 % | 0,20 % | 0,20 % | 0,20 % | 0,20 % | 0,20 % |
| B | Glycerin | 2,35 % | 2,35 % | 2,35 % | 2,35 % | 2,35 % | 2,35 % | 2,35 % |
| | Wasser | ad 100 % | ad 100 % | ad 1,50 % | ad 100 % | ad 100 % | ad 100 % | ad 100 % |
| C | NaOH (10 %) | q.s. | q.s. | q.s. | q.s. | q.s. | q.s. | q.s. |
| | Preservative, Parfüm | q.s. | q.s. | q.s. | q.s. | q.s. | q.s. | q.s. |
| | Stabilität | sehr gut | sehr gut | sehr gut | sehr gut | gut | gut | Instabil |

## Patentansprüche

1. Flüssige, kalt verarbeitbare, einphasige Öl-in-Wasser-Emulgatorsysteme, enthaltend eine flüssige, PEG-freie Emulgatorbasis bestehend aus
A) einem oder mehreren Polyolpartialestern ausgewählt aus mindestens einer der Gruppen
A1) Sorbitan- oder Sorbitolpartialester, vorzugsweise herstellbar durch Veresterung von aliphatischen, linearen oder verzweigten, ggf. ungesättigten und/oder hydroxyfunktionalisierten Carbonsäuren mit einer Kettenlänge von 6 bis 22 C-Atomen mit Sorbitol,
A2) Glycerin- und Polyglycerinpartialester, vorzugsweise herstellbar durch Veresterung von aliphatischen, linearen oder verzweigten, ggf. ungesättigten und/oder hydroxyfunktionalisierten Carbonsäuren mit einer Kettenlänge von 6 bis 22 C-Atomen mit Glycerin, Polyglycerinen oder Gemischen aus beiden,
und
B) neutralisierte Zitronensäurepartialester, deren hydrophobe Reste jeweils 10 bis 16 C-Atome enthalten
und gegebenenfalls
C) polaren flüssigen Lösungsvermittlern.

2. Flüssige, kalt verarbeitbare Öl-in-Wasser-Emulgatorsysteme gemäß Anspruch 1, **dadurch gekennzeichnet, dass** sie als flüssige Emulgatorkomponente A) Sorbitanester und Polyglycerinester enthalten.

3. Flüssige, kalt verarbeitbare Öl-in-Wasser-Emulgatorsysteme gemäß Anspruch 2, **dadurch gekennzeichnet, dass** sie als flüssige Emulgatorkomponente A) Sorbitan Laurate und Polyglyceryl-Laurate enthalten.

4. Flüssige, kalt verarbeitbare Öl-in-Wasser-Emulgatorsysteme gemäß mindestens einem der Ansprüche 1 bis 3, **dadurch gekennzeichnet, dass** die hydrophoben Estergruppen der Emulgatorkomponente A) und/oder B) Laurinsäure- bzw. Laurinalkoholreste sind.

5. Flüssige, kalt verarbeitbare Öl-in-Wasser-Emulgatorsysteme gemäß mindestens einem der Ansprüche 1 bis 4, **dadurch gekennzeichnet, dass** als flüssige polare Lösungsvermittler C) mindestens eine Verbindung ausgesucht aus der Gruppe Wasser, Glykole, Polyalkylenglykole, Glycerine, Polyglycerine, Alkohole mitverwendet werden.

6. Verwendung der flüssigen, kalt verarbeitbaren Öl-in-Wasser-Emulgatorsysteme gemäß mindestens einem der Ansprüche 1 bis 5 zur Herstellung gegebenenfalls dispergierter Feststoffe enthaltender kosmetischer, dermatologischer oder pharmazeutischer Zubereitungen.

7. Verwendung der flüssigen, kalt verarbeitbaren Öl-in-Wasser-Emulgatorsysteme nach einem der Ansprüche 1 bis 5 zur Herstellung gegebenfalls dispergierter Feststoffe enthaltender Pflege- und Reinigungsmittel für Haushalt und Industrie.

## Claims

1. Liquid, cold-processable single-phase oil-in-water emulsifier systems comprising a liquid PEG-free emulsifier base consisting of
A) one or more polyol partial esters selected from at least one of the groups
A1) sorbitan or sorbitol partial esters, preferably preparable by esterification of aliphatic, linear or branched, optionally unsaturated and/or hydroxy-functionalized carboxylic acids having a chain length of 6 to 22 carbon atoms, with sorbitol,
A2) glycerol and polyglycerol partial esters, preferably preparable by esterification of aliphatic, linear or branched, optionally unsaturated and/or hydroxy-functionalized carboxylic acids having a chain length of 6 to 22 carbon atoms, with glycerol, polyglycerols or mixtures of the two,
and
B) neutralized citric acid partial esters of which the hydrophobic residues each comprise 10 to 16 carbon atoms
and optionally
C) polar liquid solubilizers.

2. Liquid, cold-processable oil-in-water emulsifier systems according to Claim 1, **characterized in that** said systems comprise sorbitan esters and polyglycerol esters as liquid emulsifier component A).

3. Liquid, cold-processable oil-in-water emulsifier systems according to Claim 2, **characterized in that** said systems comprise sorbitan laurates and polyglyceryl laurates as liquid emulsifier component A).

4. Liquid, cold-processable oil-in-water emulsifier systems according to at least one of Claims 1 to 3, **characterized in that** the hydrophobic ester groups of emulsifier component A) and/or B) are lauric acid residues or lauryl alcohol residues.

5. Liquid, cold-processable oil-in-water emulsifier systems according to at least one of Claims 1 to 4, **characterized in that** at least one compound selected from the group of water, glycols, polyalkylene glycols, glycerols, polyglycerols, alcohols are used as liquid polar solubilizer C).

6. Use of the liquid, cold-processable oil-in-water emulsifier systems according to at least one of Claims 1 to 5 for preparing cosmetic, dermatological or pharmaceutical preparations comprising optionally dispersed solids.

7. Use of the liquid, cold-processable oil-in-water emulsifier systems according to any of Claims 1 to 5 for preparing care compositions and cleaning compositions comprising optionally dispersed solids for household and industrial use.

## Revendications

1. Systèmes d'émulsifiant huile-dans-eau liquides, transformables à froid, à une phase, contenant une base d'émulsifiant liquide, exempte de PEG, constitués par
A) un ou plusieurs esters partiels de polyol, choisis dans au moins un des groupes
A1) esters partiels de sorbitane ou de sorbitol, pouvant de préférence être préparés par estérification d'acides carboxyliques aliphatiques, linéaires ou ramifiés, le cas échéant insaturés et/ou fonctionnalisés par hydroxy, présentant une longueur de chaîne de 6 à 22 atomes de carbone avec du sorbitol,
A2) esters partiels de glycérol ou de polyglycérol, pouvant de préférence être préparés par estérification d'acides carboxyliques aliphatiques, linéaires ou ramifiés, le cas échéant insaturés et/ou fonctionnalisés par hydroxy, présentant une longueur de chaîne de 6 à 22 atomes de carbone avec du glycérol, des polyglycérols ou des mélanges des deux,
et
B) des esters partiels, neutralisés de l'acide citrique, dont les radicaux hydrophobes contiennent à chaque fois 10 à 16 atomes de carbone,
et le cas échéant
C) des promoteurs de solubilisation liquides, polaires.

2. Systèmes d'émulsifiant huile-dans-eau, liquides, transformables à froid selon la revendication 1, **caractérisés en ce qu'**ils contiennent, comme composant A) d'émulsifiant liquide, des esters de sorbitane et des esters de polyglycérol.

3. Systèmes d'émulsifiant huile-dans-eau, liquides, transformables à froid selon la revendication 2, **caractérisés en ce qu'**ils contiennent, comme composant A) d'émulsifiant liquide, des laurates de sorbitane et des laurates de polyglycéryle.

4. Systèmes d'émulsifiant huile-dans-eau, liquides, transformables à froid selon au moins l'une quelconque des revendications 1 à 3, **caractérisés en ce que** les groupes ester hydrophobes du composant A) et/ou B) d'émulsifiant sont des radicaux d'acide laurique ou d'alcool laurique.

5. Systèmes d'émulsifiant huile-dans-eau, liquides, transformables à froid selon au moins l'une quelconque des revendications 1 à 4, **caractérisés en ce qu'**on utilise conjointement, comme promoteurs de solubilisation C) liquides, polaires, au moins un composé choisi dans le groupe constitué par l'eau, les glycols, les polyalkylèneglycols, les glycérols, les polyglycérols, les alcools.

6. Utilisation des systèmes d'émulsifiant huile-dans-eau, liquides, transformables à froid selon au moins l'une quelconque des revendications 1 à 5, pour la préparation de compositions cosmétiques, dermatologiques ou pharmaceutiques contenant le cas échéant des solides dispersés.

7. Utilisation des systèmes d'émulsifiant huile-dans-eau, liquides, transformables à froid selon au moins l'une quelconque des revendications 1 à 5, pour la préparation d'agents de nettoyage et d'entretien domestiques et industriels, contenant le cas échéant des solides dispersés.
